# EUROPEAN PATENT SPECIFICATION

(11) **EP 0 675 898 B1**
(45) Date of publication and mention of the grant of the patent: **21.11.2001**
(21) Application number: 94904058.8
(22) Date of filing: 27.12.1993
(51) Int. Cl.: C07K 1/00, C07B 53/00

(54) **USE OF MOLECULARLY IMPRINTED POLYMERS FOR STEREO- AND/OR REGIOSELECTIVE SYNTHESIS**
VERWENDUNG VON MOLEKULaR GEPRÄGTEN POLYMEREN FÜR STEREO- UND/ODER REGIOSELEKTIVE SYNTHESE
UTILISATION DE POLYMERES IMPRIMES DE MANIERE MOLECULAIRE DANS LA SYNTHESE STEREOSPECIFIQUE ET/OU REGIOSELECTIVE

(30) Priority: 28.12.1992 SE 9203913
(43) Date of publication of application: 11.10.1995
(73) Proprietor: MOSBACH, Klaus H., S-244 02 Furulund (SE)
(72) Inventor: MOSBACH, Klaus, S-244 94 Furulund (SE); NICHOLLS, Ian, A., S-227 33 Lund (SE); RAMSTRÖM, Olof, S-226 47 Lund (SE)
(74) Representative: Wiklund, Erik
(86) International application number: SE9301107
(87) International publication number: WO9414835

(56) References cited:
- CHEMICAL ABSTRACTS, Volume 112, No. 5, 29 January 1990 (29.01.90), (Columbus, Ohio, USA), WULFF GUENTER et al., "Enzyme-Analog Built Polymers. 26. Enantioselective Synthesis of Amino Acids Using Polymers Possessing Chiral Cavities Obtained by an Imprinting Procedure with Template Molecules", page 640, the Abstract No. 36407h, Makromol. Chem. 1989, 190 (7), 1727-1735.
- Journal of the American Chemical Society, Volume 102, No. 9, April 1980, JULIEN DAMEN et al., "Stereoselective Syntheses via a Photochemical Template Effect", page 3265 - page 3267.
- Ekberg, B., Mosbach, K., Molecular Imprinting: A Technique for Producing Specific Separation Materials, Trends Biotechnol., 7, 92-96, 1989
- O-Shannessy, D., Ekberg, B., Andersson, L. I., Mosbach, ;., Recent Advances in the Preparation and Use of Molecularly Imprinted Polymers for Enantiomeric Resolution of Amino Acid Derivatives, J. Chromatogr., 470, 391-399, 1989
- K. Nilsson, Trends in Biotechnology, 1988, vol. 6, 256-264.

## Description

This invention refers to the use of molecularly imprinted polymers for stereo- and regioselective synthesis.

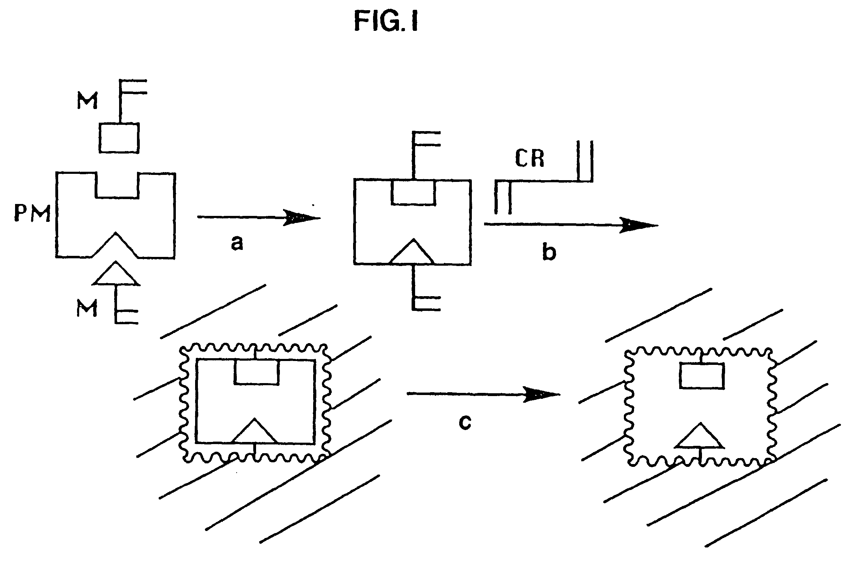

"Molecular imprinting" is the name given to a process for preparing polymers that are selective for a particular compound (the "print molecule"). The technique involves: 1) prearranging the print molecule and the monomers and allowing complementary interactions to develop; 2) polymerizing around the print molecule-monomer complex; and 3) removing the print molecule from the polymer by extraction (Fig. 1). Polymerization thus preserves the complementarity to the print molecule, and subsequently the polymer will selectively adsorb the print molecule. The print molecule binds more favourably to the extracted polymer than to structural analogues. The technique has also been referred to as "host-guest" polymerization and "template" polymerization. Preparation of the selective polymers is easy, involving only simple, well-known laboratory techniques.

J. Am. Chem. Soc. Vol. 102, 1980, 3265-3267 discloses stereoselective synthesis via a photochemical template effect. Macrom. Chem. 1989, 190(7), 1725-1735 discloses enantioselective synthesis of amino acids using polymers possessing chiral cavities obtained by an imprinting procedure with template molecules.

Usually, one of two fundamentally different approaches has been followed in applying molecular imprinting: 1) the print molecule has been covalently, but reversibly bound; or 2) the initial interactions between monomers and the print molecule have been non-covalent. The covalent approach involves, in contrast to the non-covalent approach, the cumbersome covalent bond formation between the print molecule and the monomer (polymer).

Most organic reactions are carried out in free solution, one exception being catalysts immobilized on a solid matrix. Another example is the formation or sequencing of macromolecules such as peptides or polynucleotides following the so-called Merrifield approach where synthetic reactions are taking place on solid phase.

One disadvantage in using conventional solution chemistry is: Since several reactive groups in e.g. condensation reactions can often be involved, a great number of isomers, may they be regio- or stereoisomers, can be formed. To avoid the latter complications, various strategies of protecting such functional groups are being used.

The event of molecular imprinting involving, as described above, the formation of specific imprints (e.g. regio- and/or stereoselective) allows in principle synthetic reactions to take place in the cavities formed. The cavities will thus direct the synthesis in the desirable direction. In addition, it is possible that the surrounding polymer matrix will "take over" the function of the protecting groups. An additional fringe benefit of the approach is the fact that, because the cavities are specific, crude samples can be used, whereby the desired reaction products in a polymer matrix can subsequently be specifically isolated.

Furthermore, the repeated use of the polymer matrix is of great potential advantage and isolation of the products are made easier.

It is now a well established technique to mix an imprint molecule with monomers and crosslinkers followed by their polymerization around the imprint molecule and extraction of the latter. The monomers of often different functionalities interact during imprinting as well as subsequent recognition by non-covalent interactions such as ion-pair formation, dipolar electrostatic interactions, hydrogen bonding, charge transfer interactions and metal coordination (2, 3, 4). Alternatively, covalent interactions between imprint molecule and polymerizable monomers can be used (1). The most widely used monomers include various acrylates, heteroaromatics and vinylbenzenes. Such imprints can, according to the present invention, be used for chemical synthesis.

The additional aspect of using such imprints for catalysis, i.e. involving turnover, is an area of potential interest which however requires a number of prerequisites to be fulfilled such as the correct positioning of the catalyst or penetration of the catalyst through the polymer as well as easy dissociation of the formed products.

### Short description of the drawings:

Fig. 1 shows the principle of molecular imprinting.

Fig. 2 shows the use of molecularly imprinted polymers for the selective removal of one enantiomeric or regio-isomeric species from solution, while reaction takes place with the antipode or regio-isomer in bulk solution.

Fig. 3 shows molecularly imprinted polymers used as interacting protecting matrix.

Fig. 4 shows the selective benzoylation of a carbohydrate derivative,

Fig. 5 shows the use of molecularly imprinted polymers for directed synthesis.

Fig. 6 shows the site-specific coupling of N-protected L-tryptophanyl cloride to DL-phenylalanine methyl ester.

Fig. 7 shows the chemoselective synthesis of N-protected amino acids.

Fig. 8 shows the combined use of the direction and protection strategies.

Further to the above, Fig. 1 shows the development of complementary interactions between the print molecule and the monomers (a); polymerization (b); removal of the print molecule from the polymer (c). M = monomers, PM = print molecule, CR = crosslinker.

One aspect of the invention describes the use of molecularly impinted polymers for the selective removal of one enantiomeric or regio-isomeric species from solution, while reaction takes place with the enantiopode or regio-isomer in bulk solution, in the synthesis of peptides, oligosaccarides and oligonucleotides (5). This is achieved by the preparation of suitable imprinted polymer, careful manipulation of reaction stoichiometry and selection of suitable condensation reagents.

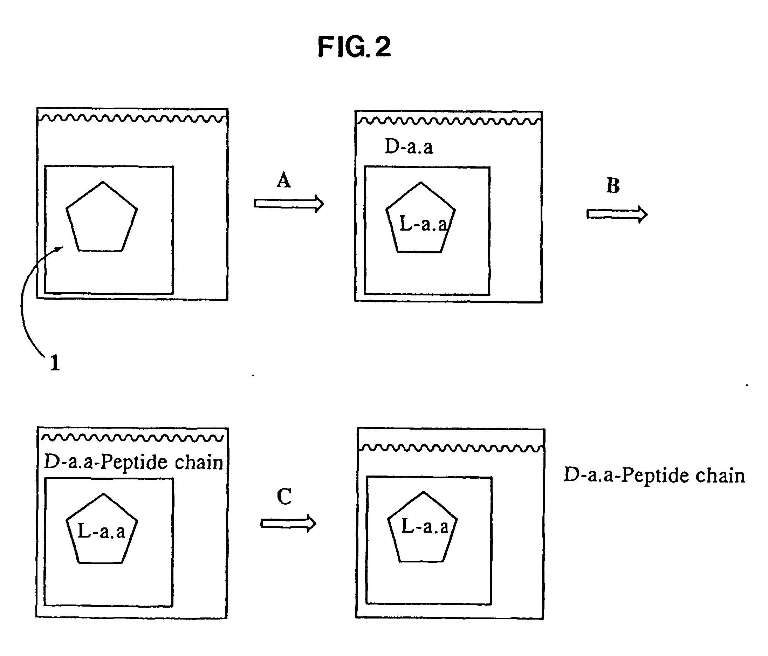

More particularly, this aspect relates to the use of a molecularly imprinted polymer for selective removal of one enantiomeric or regio-isomeric species from a bulk solution during synthesis of peptides, oligosaccharides or oligonucleotides, whereby
one enantiomeric or regio-isomeric template or imprint species is non-covalently imprinted in the polymer,
the template or imprint species is extracted,
the polymer is incubated with a bulk solution of a racemic mixture of the enantiomeric or regio-isomeric species, leading to selective enrichment, by non-covalently interactions, of said on enantiomeric or regio-isomeric species in the polymer and the other enantiomeric or regio-isomeric species in the bulk solution,
whereafter the other enantiomeric or regio-isomeric species is reacted in the bulk solution. This aspect is outlined in Fig. 2, in which 1 symbolizes a molecularly imprinted polymer against, in this case, an L-enantiomer of an amino acid or amino acid derivative. The polymer in solution is incubated with the racemic mixture of the amino acid or amino acid derivative during step A leading to the selective enrichment, by non-covalent interactions, of the L-enantiomer in the polymer and the D-enantiomer in solution. A suitable coupling reagent together with the adequately protected amino acid or peptide chain are introduced in step B. After coupling with the D-enantiomer in solution, the peptide chain is isolated by filtration in step C, whereas the L-enantiomer remains primarily in the polymer throughout the whole process.

Example 1 below describes the enantioselective synthesis of a dipeptide, N-acetyl-D-tryptophanyl-L-phenyl-alanin methyl ester, utilizing a polymer imprinted against N-acetyl-L-tryptophan and, after incubation with the racemic mixture thereof, subsequent condensation with L-phenylalanine methyl ester in the bulk solution.

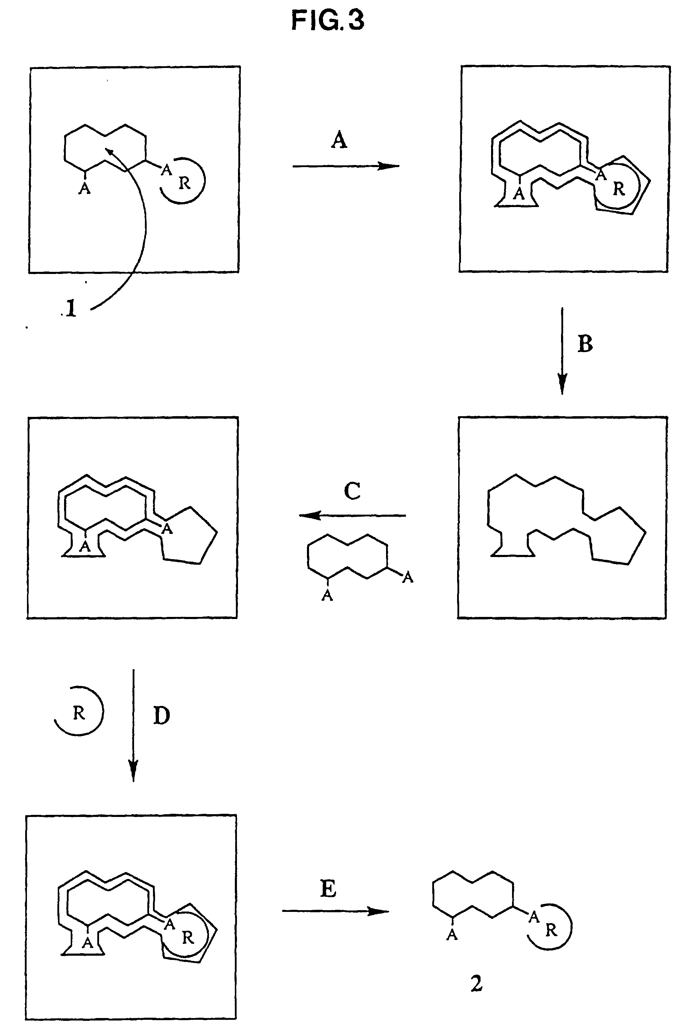

Another aspect of the invention covers the use of such imprinted polymers to act as interaction protecting matrices, capable of regio-selectively preventing a particular reaction at a specific site of a molecule containing several potentially reactive sites. This aspect is outlined in Fig. 3. A carefully selected substrate (1) incorporating two reactive sites (A), selectively protected in one position by a protecting group (R), is imprinted by non-covalent interactions in the designed polymer during step A. Exhaustive extraction of the polymer during step B leaves the polymer with complementary imprints of 1. Following incubation of the non-protected substrate analogue in step C, addition of a protecting group reagent (R or R') in step D leads to site-specific reaction with the free functional group (A). Finally, the product is isolated by extraction of the polymer in step E.

It is conceived that, for example, in the case of peptide synthesis, the following groups can be substituted by interaction with the imprinted polymer matrix, may it be by complementary binding or by the surrounding matrix per se:

| Protection of functional group | Protecting group |
|---|---|
| Amino | tert-butyloxycarbonyl (BOC) |
| | 9-fluorenylmethyloxycarbonyl (Fmoc) |
| | benzyloxycarbonyl (Cbz) |
| | biphenylisopropyloxycarbonyl (Bpoc) |
| Carboxyl | methyl |
| | tert-butyl |
| | benzyl |
| Hydroxyl | tert-butyl |
| | benzyl |
| Thiol | benzyl |
| | acetimidomethyl (Acm) |
| Guanidino | nitro |
| | tosyl |
| | adamantyloxycarbonyl (Adoc) |
| Imidazol | benzyloxymethyl (BOM) |

The same concept should be valid also for synthesis of other compounds such as carbohydrates, nucleotides, etc.

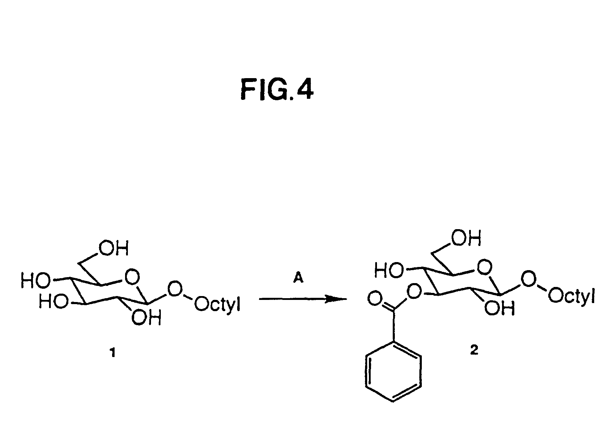

One example of this aspect is e.g. the selective benzoylation of a carbohydrate derivative utilizing selective protection of hydroxy groups by binding of the carbohydrate derivative to an imprinted polymer matrix, as indicated in Fig. 4. In this example, a polymer is imprinted against octyl-3-O-benzoyl-β-D-glucopyranoside (2) resulting in sites complementary to the above compound with the surrounding polymer matrix serving as protecting "agent" at the 2-O-, 4-O-, and 6-O-positions. Incubation of these polymers with octyl-β-D-glucopyranoside (1) and subsequent reaction with a suitable benzoylating agent (A) renders preferably the 3-O-benzoyl-derivative in favour of the 2-O-, 4-O-, or 6-O-derivatives.

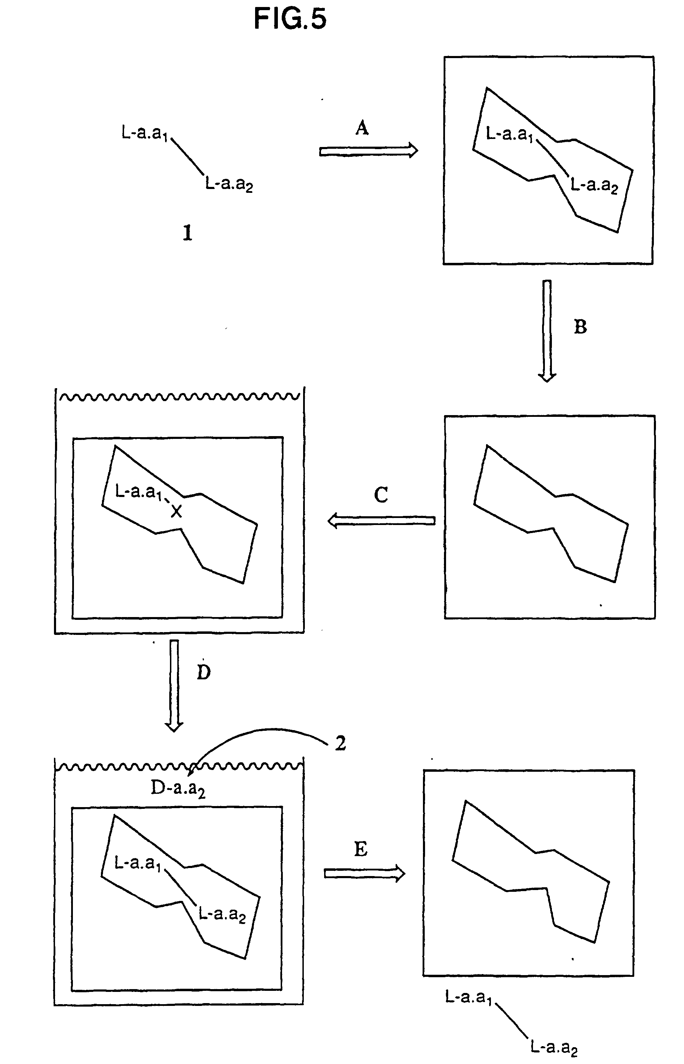

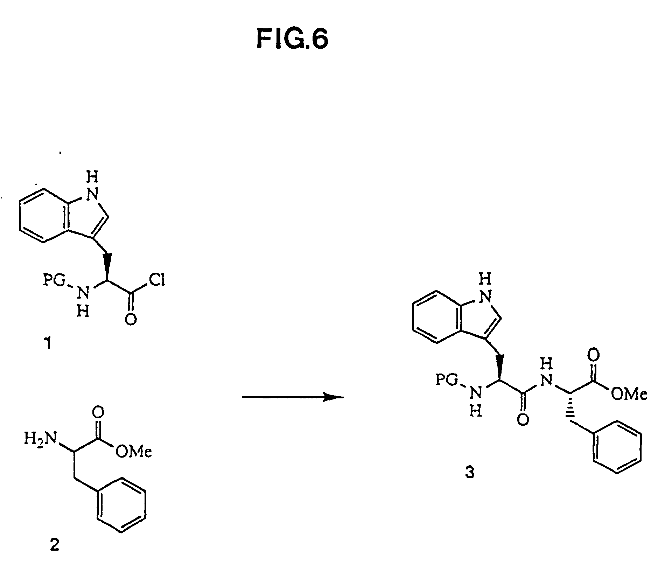

A third aspect of the present invention covers the use of such imprinted polymers for directed synthesis such as enantioselective synthesis of peptides, whereby condensation of amino acid (derivatives) is allowed to take place in the preformed recognition cavity. This aspect is described in Fig. 5. A carefully selected template or imprint species, in this case a suitably protected dipeptide (1), is imprinted by non-covalent interactions during step A. Extraction of the template in step B, results in a polymer matrix containing complementary recognition sites for 1. An activated form of amino acid 1 (L-a.a₁-X) is incubated in the polymeric sites during step C and addition of a racemic mixture of the second amino acid (DL-a.a₂) during step D leads to specific condensation in the cavity of the L-enantiomer forming the dipeptide corresponding to the template species (1). The resulting product is finally isolated by extraction in step E. An example of this strategy is e.g. the site-specific coupling of N-protected L-tryptophanyl chloride to DL-phenylalanine methyl ester (Fig. 6) where PG represents a protecting group such as e.g. a benzyloxycarbonyl (Cbz) group. In this example, a polymer is imprinted against the N-protected dipeptide N-PG-L-tryptophanyl-L-phenylalanine methyl ester (3) leading to the formation of sites complementary in shape and functionality to this imprint molecule. Incubation of the polymer with N-PG-tryptophanyl chloride (1) followed by addition of DL-phenylalanine methyl ester (2) effects the preferential synthesis of the imprint species (3), thus minimizing formation of N-PG-L--tryptophanyl-D-phenylalanine methyl ester.

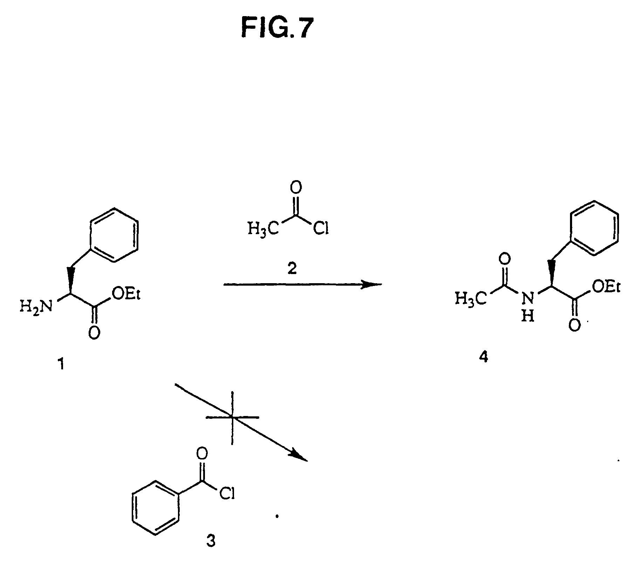

Another example of this aspect is the chemoselective synthesis of N-protected amino acids as outlined in Fig. 7. A polymer is imprinted against N-acetyl-L-phenylalanine ethyl ester (4) resulting in recognition sites complementary to this particular N-acetylated amino acid ester. Incubation of the polymer matrix with L-phenylalanine ethyl ester (1) and subsequent addition of either acetyl chloride (2) or benzoyl chloride (3) leads to preferential formation of the imprinted molecule (4), whereas the formation of the benzoylated derivative is inhibited. In a mixture of the acylating reagents, polymer-assisted formation of a high yield of N-acetyl-L-phenylalanine ethyl ester is obtained as compared to N-benzoyl-L-phenylalanine ethyl ester.

An additional example along these lines is the practically useful regio-selective synthesis of triglycerides from glycerol and various fatty acids. In this case, molecularly imprinted polymers can be used to direct the specific condensation of certain fatty acids with the glycerol moiety in order to obtain a required triacylglyceride.

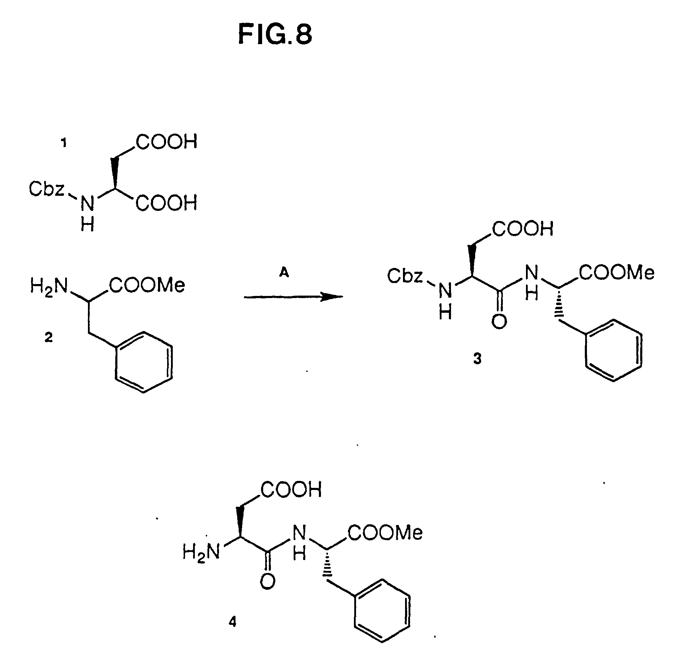

An example of employing molecularly imprinted polymers for applications combining both the direction and protection strategies is outlined in Fig. 8. For instance, these polymers can be imprinted against derivatives of molecules originally containing two or more identical functional groups, e.g. the dipeptide N-benzyloxycarbonyl--L-aspartyl-L-phenylalanine methyl ester (3). The β-carboxy group of this template species is unprotected and the carboxy group in the α-position of the aspartic acid residue is coupled to the phenylalanine residue. The resulting polymer leaves specific enantio- and regioselective interaction sites for the template molecule serving as a protecting matrix for the β-carboxy group. Incubation of the polymer with N-benzyloxycarbonyl-L-aspartic acid (1) and subsequent addition of DL-phenylalanine methyl ester (2) under suitable coupling conditions such as with reagents (A) renders preferably the α-dipeptide, whereas the β-isomer cannot be formed. Furthermore, as the imprint was prepared against the L-form of the second amino acid, preferential coupling with the L-form will occur in the cavity. Subsequent removal of the Cbz-group leads to the formation of the industrially important sweetening agent α-aspartame (4).

Another example is found in the area of conversion of antibiotics. For instance, in cavities obtained from cephalosporin C, selective cleavage of the side-chain leading to the useful 7-aminocephalosporanic, 7-ACA, can take place, alternatively similar imprints can be used for directed synthesis of semisynthetic cephalosporins from 7-ACA.

Another example utilizing both the surrounding polymer matrix as substitute for protecting groups, especially hydroxyl groups, and directing the synthesis is to be found in the syntheses of carbohydrates such as disaccharides. In one case imprinting of the disacharide 4-O-(β-D--galactopyranosyl)-β-D-2-deoxy-2-(N-acetylamino)-glucopyranose is followed by extraction. Subsequent condensation in the cavities of D-galactose and N-acetyl-D-glucosamine leads to the original imprint molecule. Analogously the important compound metyl-3-O-(β-D-galactopyranosyl)--β-D-glucopyranoside can be synthesized in a similar fashion. The condensation could be carried out following the Fischer reaction using solvents saturated with gaseous HCl or by utilizing one activated monosaccharide obtained by bromination at the anomeric carbon (6).

### Example 1

In a typical experiment, a molecularly imprinted polymer was prepared against N-acetyl-L-tryptophan. Racemic N-acetyl-tryptophan (10 mg/ml in dry dimethylformamide) was incubated overnight at 4°C in the presence of the imprinted polymer (500 mg) in a total volume of 2 ml, made up with tetrahydrofuran. After cooling to 0°C, L-phenylalanine metyl ester (1 eq.) and 1-hydroxybenzotriazole (1.1 eq) were added, followed by N,N'-dicyclohexylcarbodiimide (1.1 eq.). The reaction mixture was allowed to stand for 24 h, at room temperature, then filtered and the residue washed successively with portions of tetrahydrofuran and methanol/acetic acid. The filtrate was concentrated to dryness in vacuo and the residue partitioned between ethyl acetate and saturated sodium bicarbonate. The organic phase was successively washed with aqueous citric acid, saturated sodium bicarbonate and water, then dried and concentrated in vacuo. The crude products were purified by preparative thin layer chromatography, isolated and analysed by nuclear magnetic resonance (NMR). 36% diastereomeric excess of N-acetyl-D-tryptophanyl-L-phenylalanine methyl ester over N-acetyl-L--tryptophanyl-L-phenylalanine methyl ester was obtained.

### REFERENCES

1. Ekberg, B., Mosbach, K. Molecular Imprinting: a Technique for Producing Specific Separation Materials, Trends Biotechnol., 7, 92-96, 1989.
2. O'Shannessy, D., Ekberg, B., Andersson, L. I., Mosbach, K. Recent Advances in the Preparation and Use of Molecularly Imprinted Polymers for Enantiomeric Resolution of Amino Acid Derivatives, J. Chromatogr., 470, 391-399, 1989.
3. Ramström, O., Andersson, L. I., Mosbach, K., Recognition Sites Incorporating both Pyridinyl and Carboxy Functionalities Prepared by Molecular Imprinting, J. Org. Chem., in press
4. Ramström, O., Nicholls, I. A., Mosbach, K., Synthetic Polymer peptide receptors: Stereoselective Recognition in Non-Covalent Molecularly Imprinted Polymers, Tetrahedron:Asymmetry, submitted for publication.
5. Nicholls, I. A., Ramström, O., Mosback, K. Enantioselective Mediation of Reactivity by Molecularly Imprinted Polymer Derived Antibody Combining Site Mimics, manuscript.
6. K. Nilsson, Trends in Biotechnology 1988, Vol 6, 256-264.

## Claims

1. Use of a molecularly imprinted polymer for selective removal of one enantiomeric or regio-isomeric species from a bulk solution during synthesis of peptides, oligosaccharides or oligonucleotides, whereby
one enantiomeric or regio-isomeric template or imprint species is non-covalently imprinted in the polymer,
the template or imprint species is extracted,
the polymer is incubated with a bulk solution of a racemic mixture of the enantiomeric or regio-isomeric species, leading to selective enrichment, by non-covalently interactions, of said one enantiomeric or regio-isomeric species in the polymer and the other enantiomeric or regio-isomeric species in the bulk solution,
whereafter the other enantiomeric or regio-isomeric species is reacted in the bulk solution.

2. Use according to claim 1, whereby said one enantiomeric or regio-isomeric species is N-acetyl-L- or D-tryptophan, and said other enantiomeric or regio-isomeric species is reacted with L- or D-phenylalanine methyl ester, respectively, in the bulk solution, resulting in the formation of N-acetyl-D- or -L-tryptophanyl-L-phenylalanin methyl ester or N-acetyl-D- or -L-tryptophanyl-D-phenylalanin methyl ester, respectively.

3. Use of a molecularly imprinted polymer for stereo- and regioselective synthesis, whereby
a regioselectively interacting protecting polymer matrix, prepared by non-covalently imprinting of a molecule comprising several potentially reactive sites of which one is selectively protected and extracting the molecule leaving a polymer having complementary imprints of the molecule, is incubated with a non-protected analogue of the molecule,
a protective group reagent is added leading to site-specific reaction, and
the molecule is isolated by extracting of the polymer.

4. Use according to claim 3 for selective benzoylation of a carbohydrate derivative, whereby hydroxy groups on the carbohydrate derivative are selectively protected by binding of the carbohydrate derivate to an imprinted polymer matrix.

5. Use according to claim 4, wherein the carbohydrate derivative is octyl-β-D-glucopyranoside.

6. Use of a molecularly imprinted polymer for enantioselective synthesis, whereby
an enantiomeric template or imprint species is non-covalently imprinted in the polymer,
the template or imprint species is extracted,
an activated form of one part of the enantiomeric template or imprint species is incubated in the imprinted polymer,
and a racemic mixture of the other part of the enantiomeric template is added,
leading to a specific condensation in the imprint of an enantiomer corresponding to the template or imprint species.

7. Use according to claim 6, whereby the enantiomeric template or imprint species is a protected dipeptide, the activated form of one part of the enantiomeric template or imprint species is a first amino acid, and the racemic mixture of the other part of the enantiomeric template or imprint is a racemic mixture of a second amino acid.

8. Use according to claim 7, whereby the activated form of one part of the dipeptide is N-protected L-tryptophanyl chloride and the racemic mixture of the other part of dipeptide is D,L-phenylalanine methyl ester.

9. Use of a molecularly imprinted polymer for chemoselective synthesis, whereby
a template or imprint species carrying a specific group or moiety is non-covalently imprinted in the polymer,
the template or imprint species is extracted, resulting in recognition sites in the polymer complementary to the template or imprint species,
whereafter the template or imprint species devoid of said group or moiety is incubated in the imprinted polymer, and
a group or moiety constituting part of the template or imprint species is added, optionally in admixture with other groups or moieties having the same chemical reactivity but different forms or sizes,
leading to a specific condensation in the imprint of the group corresponding to the template or imprint species.

10. Use according to claim 9, whereby the template or imprint species is N-acetyl-L-phenylalanine ethyl ester and the imprinted polymer is incubated with L-phenylalanine ethyl ester and acetyl or benzoyl chloride.

11. Use of molecularly imprinted polymers for stereo- and regioselective synthesis utilizing the combined functions of imprints serving as protecting matrices, as claimed in claim 3, and acting as templates for directing reactions, as claimed in claim 1, 6 or 9.

## Patentansprüche

1. Verwendung eines molekular geprägten Polymers zur selektiven Entfernung einer enantiomeren oder regioisomeren Spezies aus einer Gesamtlösung während der Synthese von Peptiden, Oligosacchariden oder Oligonucleotiden, wobei
eine enantiomere oder regioisomere Templat- oder Prägespezies nichtkovalent in das Polymer eingeprägt wird,
die Templat- oder Prägespezies extrahiert wird,
das Polymer mit einer Gesamtlösung eines racemischen Gemisches der enantiomeren oder regioisomeren Spezies inkubiert wird, was zu einer selektiven Anreicherung, durch nichtkovalente Wechselwirkungen, der einen enantiomeren oder regioisomeren Spezies in dem Polymer und der anderen enantiomeren oder regioisomeren Spezies in der Gesamtlösung führt,
wonach die andere enantiomere oder regioisomere Spezies in der Gesamtlösung umgesetzt wird.

2. Verwendung nach Anspruch 1, wobei die eine enantiomere oder regioisomere Spezies N-Acetyl-L- oder D-Tryptophan ist, und die andere enantiomere oder regioisomere Spezies mit L- oder D-Phenylalaninmethylester in der Gesamtlösung umgesetzt wird, was zur Bildung von N-Acetyl-D- oder -L-tryptophanyl-L-phenyl-alaninmethylester bzw. N-Acetyl-D- oder -L-tryptophanyl-D-phenylalaninmethylester führt.

3. Verwendung eines molekular geprägten Polymers für eine stereo- und regioselektive Synthese, wobei
eine regioselektiv wechselwirkende Schutzpolymermatrix, die durch nichtkovalentes Einprägen eines Moleküls, das mehrere potentiell reaktive Stellen umfasst, von denen eine selektiv geschützt ist, und Extrahieren des Moleküls unter Zurücklassung eines Polymers mit komplementären Einprägungen bzw. Abdrücken des Moleküls hergestellt wird, mit einem ungeschützten Analogon des Moleküls inkubiert wird,
ein Schutzgruppenreagenz zugegeben wird, was zu einer regiospezifischen (ortsspezifischen) Reaktion führt, und
das Molekül durch Extrahieren des Polymers isoliert wird.

4. Verwendung nach Anspruch 3 zur selektiven Benzoylierung eines Kohlenhydratderivats, wobei Hydroxygruppen an dem Kohlenhydratderivat durch Binden des Kohlenhydratderivats an eine geprägte Polymermatrix selektiv geschützt werden.

5. Verwendung nach Anspruch 4, wobei das Kohlenhydratderivat Octyl-β-D-glucopyranosid ist.

6. Verwendung eines molekular geprägten Polymers für eine enantioselektive Synthese, wobei
eine enantiomere Templat- oder Prägespezies nichtkovalent in das Polymer eingeprägt wird,
die Templat- oder Prägespezies extrahiert wird,
eine aktivierte Form von einem Teil der enantiomeren Templat- oder Prägespezies in dem geprägten Polymer inkubiert wird,
und ein racemisches Gemisch des anderen Teils des enantiomeren Templats zugegeben wird,
was zu einer spezifischen Kondensation in der Einprägung bzw. dem Abdruck eines Enantiomers entsprechend der Templat- oder Prägespezies führt.

7. Verwendung nach Anspruch 6, wobei die enantiomere Templat- oder Prägespezies ein geschütztes Dipeptid ist, die aktivierte Form von einem Teil der enantiomeren Templat- oder Prägespezies eine erste Aminosäure ist, und das racemische Gemisch des anderen Teils des enantiomeren Templats oder Abdrucks ein racemisches Gemisch einer zweiten Aminosäure ist.

8. Verwendung nach Anspruch 7, wobei die aktivierte Form von einem Teil des Dipeptids N-geschütztes L-Tryptophanylchlorid ist und das racemische Gemisch des anderen Teils des Dipeptids D,L-Phenylalaninmethylester ist.

9. Verwendung eines molekular geprägten Polymers für eine chemoselektive Synthese, wobei
eine Templat- oder Prägespezies, die eine spezifische Gruppe oder Komponente trägt, nichtkovalent in das Polymer eingeprägt wird,
die Templat- oder Prägespezies extrahiert wird, was zu Erkennungsstellen in dem Polymer führt, die zu der Templat- oder Prägespezies komplementär sind,
wonach die Templat- oder Prägespezies ohne diese Gruppe oder Komponente in dem geprägten Polymer inkubiert wird, und
eine Gruppe oder Komponente, die einen Teil der Templat- oder Prägespezies bildet, zugegeben wird, gegebenenfalls in Mischung mit anderen Gruppen oder Komponenten mit der gleichen chemischen Reaktivität, aber verschiedenen Formen oder Größen,
was zu einer spezifischen Kondensation in der Einprägung bzw. dem Abdruck der Gruppe entsprechend der Templat- oder Prägespezies führt.

10. Verwendung nach Anspruch 9, wobei die Templat- oder Prägespezies N-Acetyl-L-phenylalaninethylester ist und das geprägte Polymer mit L-Phenylalaninethylester und Acetyl- oder Benzoylchlorid inkubiert wird.

11. Verwendung von molekular geprägten Polymeren für eine stereo- und regioselektive Synthese unter Verwendung der kombinierten Funktionen von Einprägungen bzw. Abdrücken, die als Schutzmatrizes dienen, wie in Anspruch 3 beansprucht, und als Template zum Lenken von Reaktionen wirken, wie in Anspruch 1, 6 oder 9 beansprucht.

## Revendications

1. Utilisation d'un polymère imprimé de manière moléculaire pour l'élimination sélective d'une espèce énantiomère ou régio-isomère d'une solution en vrac pendant la synthèse de peptides, d'oligosaccharides ou d'oligonucléotides, **caractérisée en ce que**
une espèce modèle ou imprimée énantiomère ou régio-isomère est imprimée de manière non covalente dans le polymère,
l'espèce modèle ou imprimée est extraite,
le polymère est incubé avec une solution en vrac d'un mélange racémique de l'espèce énantiomère ou régio-isomère, conduisant à un enrichissement sélectif, par des interactions non covalentes, de ladite une espèce énantiomère ou régio-isomère dans le polymère et l'autre espèce énantiomère ou réglo-Isomère dans la solution en vrac,
puis l'autre espèce énantiomère ou régio-isomère est mise à réagir dans la solution en vrac.

2. Utilisation selon la revendication 1, **caractérisée en ce que** ladite une espèce énantiomère ou régio-isomère est le N-acétyl-L- ou D-tryptophane, et ladite autre espèce énantiomère ou régio-isomère est mise à réagir avec l'ester méthylique de la L- ou D-phénylalanine, respectivement, dans la solution en vrac, ce qui a pour résultat la formation de l'ester méthylique de la N-acétyl-D- ou -L-tryptophényl-L-phénylalanine ou l'ester méthylique de la N-acétyl-D- ou -L-tryptophanyi-D-phénylalanine, respectivement.

3. Utilisation d'un polymère imprimé de manière moléculaire pour la synthèse stéréo- et régio-sélective, **caractérisée en ce que**
une matrice de polymère protégeant interagissant de manière régio-sélective, préparée par impression non covalente d'une molécule comprenant plusieurs sites réactifs potentiellement dont l'un est protégé sélectivement et par extraction de la molécule laissant un polymère ayant des impressions complémentaires de la molécule, est mise à incuber avec un analogue non protégé de la molécule,
un réactif de groupe protecteur est ajouté conduisant à une réaction spécifique sur un site, et
la molécule est isolée par extraction du polymère.

4. Utilisation selon la revendication 3 pour la benzoylation sélective d'un dérivé de glucide, **caractérisée en ce que** des groupes hydroxy sur le dérivé de glucide sont protégés sélectivement par liaison du dérivé de glucide à une matrice polymère imprimée.

5. Utilisation selon la revendication 4, **caractérisée en ce que** le dérivé de glucide est l'octyl-β-D-glucopyranoside.

6. Utilisation d'un polymère imprimé de manière moléculaire pour la synthèse énantiosélective, **caractérisée en ce que**
une espèce modèle ou imprimée énantiomère est imprimée de manière non covalente dans le polymère,
l'espèce modèle ou imprimée est extraite,
une forme active d'une partie de l'espèce modèle ou imprimée énantiomère est incubée dans le polymère imprimé, et
un mélange racémique de l'autre partie du modèle énantiomère est ajouté,
conduisant à une condensation spécifique dans l'impression d'un énantiomère correspondant à l'espèce modèle ou imprimée.

7. Utilisation selon la revendication 6, **caractérisée en ce que** l'espèce modèle ou imprimée énantiomère est un dipeptide protégé, la forme activée d'une partie de l'espèce modèle ou imprimée énantiomère étant un premier acide aminé, et le mélange racémique de l'autre partie de l'espèce modèle ou imprimée énantiomère étant un mélange racémique d'un second acide aminé.

8. Utilisation selon la revendication 7, **caractérisée en ce que** la forme activée d'une partie du dipeptide est le chlorure de L-tryptophanyle N-protégé et le mélange racémique de l'autre partie du dipeptide est l'ester méthylique de la D,L-phénylalanine.

9. Utilisation d'un polymère imprimé de manière moléculaire pour la synthèse chimisélective, **caractérisée en ce que**
une espèce modèle ou imprimée portant un groupe ou un groupement spécifique est imprimée de manière non covalente dans le polymère,
l'espèce modèle ou imprimée est extraite, ce qui a pour résultat la reconnaissance de sites dans le polymère de manière complémentaire à l'espèce modèle ou imprimée,
puis l'espèce modèle ou imprimée dépourvue dudit groupe ou groupement est incubée dans le polymère imprimé, et
un groupe ou groupement constituant une partie de l'espèce modèle ou imprimée est ajoutée, en option en mélange avec d'autres groupes ou groupements ayant la même réactivité chimique mais de tailles ou de formes différentes,
conduisant à une condensation spécifique dans l'impression du groupe correspondant à l'espèce modèle ou imprimée.

10. Utilisation selon la revendication 9, **caractérisée en ce que** l'espèce modèle ou imprimée est l'ester éthylique de la N-acétyl-L-phénylalanine et le polymère imprimé est incubé avec l'ester éthylique de la L-phénylalanine et le chlorure d'acétyle ou de benzoyle.

11. Utilisation de polymères imprimés de manière moléculaire pour la synthèse stéréo- et régio-sélective utilisant les fonctions combinées d'impressions servant de matrices protectrices, telle que revendiquée dans la revendication 3, et agissant comme modèles pour diriger les réactions, tel que revendiqué dans les revendications 1, 6 ou 9.
